# EUROPEAN PATENT APPLICATION

(11) **EP 2 042 092 A1**
(43) Date of publication of application: **01.04.2009**
(21) Application number: 07788637.2
(22) Date of filing: 29.06.2007
(51) Int. Cl.: A61B 5/04, A61N 1/05, A61N 5/08

(54) **ELECTROPHYSIOLOGICAL SENSOR**

(30) Priority: 19.07.2006 ES 200601997
(71) Applicant: Starlab Barcelona SL, 08035 Barcelona (ES)
(72) Inventor: RUFFINI, Giulio, E-08035 Barcelona (ES); DUNNE, Stephen, E-08035 Barcelona (ES); FARRÉS, Esteve, E-08035 Barcelona (ES)
(74) Representative: Gislon, Gabriele
(86) International application number: PCT/ES2007/000390
(87) International publication number: WO 2008/009761

(57) **Abstract**

The sensor comprises a plurality of conducting nanostructures (2) which can transmit an electrical signal captured from the skin or from another part of an organic tissue to a transmitter means (4, 7) for transmitting said signal, wherein said nanostructures adopt a rigid filiform configuration and are connected at one end to a conducting substrate (3) coupled to said transmitter means (4, 7), said nanostructures being operable to penetrate, by their free end, in said organic tissue (6), as needles.

## Description

### Field of the Invention

The present invention generally relates to an electrophysiological sensor, - i.e., an electrode assembly for electrophysiological applications - and particularly to a sensor adapted for electrophysiological applications which do not require conducting substances acting as an interface between the sensor and the signal taking area.

The invention likewise relates to an electrophysiological sensor operating in dry conditions particularly applicable to transmitting weak electrical signals (such as biological potential or biopotential signals) with low noise, through the skin, based on nanotechnology.

### Background of the Invention

The demand for electrophysiological sensors applied on the skin is currently increasing in modem clinics and in biomedical applications.

Patent application EP-A-1596929 describes a medical device comprising an electrode incorporating a material based on carbon nanotubes and a method for manufacturing the device.

Carbon nanotubes were discovered in approximately 1991 and from then numerous and very interesting applications such as power storage for batteries, capacitors, etc. have been found for them. The previously mentioned patent application starts from the knowledge of these nanotubes for constructing a medical device. Said medical device is used for perceiving and communicating electrical stimuli in a way similar to that considered in the present invention, but in that patent application it is necessary to apply, in at least one portion of the surface of the medical device, an adhesive layer formed by a conductive polymer for adhering the nanotubes to at least one portion of the electrode. This layer is not necessary in the sensor of the present invention, being able to use techniques such as CVD (Chemical Vapor Deposition) for the direct growth of the nanotubes on a conducting substrate in the device.

The medical device described in patent application EP-A-1596929 does not describe nor suggest that the nanotubes arranged on a portion of the electrode are configured and/or adapted for directly penetrating the skin, it must be deduced therefrom that its application will be the conventional one by means of placing an interface layer between the electrode and the skin, generally a gel type interface layer,.

The need to apply an interface layer between the electrode or sensor and the skin or another organic tissue involves some drawbacks: it is necessary to invest a long time preparing the skin and the device (in the order of a few minutes) for each intervention. The gel-skin interface is not a stable interface, which adds noise (electrode-skin, electrode-polymer or gel, gel-skin) to the measurements taken, disrupting its reliability. The conductive gel can further undergo modifications such as drying out (at least to a certain extent) during the process and therefore adding even more noise to the involved information. It is likewise necessary to act on the skin on which the device will be placed by slightly scratching or scraping the epidermal surface in order to unify it and ensure the conductivity with the consequent discomfort for the patient and increase in operating time.

Due to the foregoing, it is necessary to offer an alternative to the state of the art alleviating the discomfort generated in the patient, saving time for the operator, and reducing the disturbances and errors derived from the noise added to the measurements. It is therefore of interest to provide a sensor improving the previously indicated current deficiencies.

The electrophysiological sensor provided by the present invention provides a particular solution to this need and unlike the device described in the mentioned patent application EP-A-1596929, in the sensor provided by the invention it will be possible to dispense with the mentioned substrate-nanotube adhesive layer, furthermore adding a different operation based on penetrating the skin by the nanotubes, as is described below.

### Description of the Invention

The present invention relates to an electrophysiological electrode assembly or sensor which is basically integrated by an electrode for the skin which can be used in multiple applications such as: electroencephalograms (EEG), electrocardiograms (ECG), electro-oculography (EOG), or electromyography (EMG), among others. The sensor is based on technology with nanostructures and is applied in the transmission of weak electrical signals, with low noise, through the skin. It consists of an assembly formed by a structure of carbon nanotubes (CNT) with a particular arrangement and configuration especially favorable for electrophysiological applications.

More specifically, the electrophysiological sensor provided is of the type comprising a plurality of conducting nanostructures which can transmit a weak electrical signal captured from the skin or from another part of an organic tissue to a transmitter means formed for example by an electrical connector which continues in a conductor or wiring and is characterized by integrating a structure of multiple carbon nanotubes fixed to a suitable conducting support substrate, said nanotubes emerging from said substrate in the form of substantially rigid and filiform elements, like needles. The fixing does not require a polymer layer for adhering the nanotubes. These rigid and filiform nanostructures are thus chemically connected at one end to said conducting substrate linked to an electrical connector, and are operable to at least partially penetrate, by their free end, in said organic tissue or skin, like needles, without needing any intermediate gel or interface, i.e., the electrophysiological sensor operates in dry conditions on the skin which must not have been previously subjected to a previous preparation treatment. The contact and partial penetration of the nanotubes in the Stratum Corneum (outer layer of the epidermis with a thickness of 10 to 20 µm and forming a resistive medium) allow a stable electrical contact with low impedance and noise.

In a first embodiment the electrophysiological sensor of the invention comprises an envelopment casing housing said connector and which has associated to an outer wall the mentioned conducting substrate supporting the nanostructures.

According to a second preferred embodiment it has been provided that the sensor includes a local amplifier in association with said connector.

In an even more improved third version the sensor additionally includes a circuit for a treatment (pre-processing or processing) or at least partial adaptation of the weak electrical signals captured from the skin.

In an even more improved fourth version, the incorporation of an electronic circuit for transmitting the data captured by the sensor, by radiofrequency, to a remote management point has been provided.

The transmission and control of the electronic circuit associated to sensor is carried out wirelessly being able to couple, if necessary, other digital communication devices, as can be the case of a USB communications port.

The sensor will generally incorporate a digital electronic circuit carrying out signal conditioning, automatic gain control, automatic drift compensation, digitization and digital filtering functions.

These functions allow considerably reducing the interferences of the signal and form a substantial improvement with respect to the state of the art.

Another function of the mentioned electronic circuit is the possibility of compressing the digitized signals, prior to transmitting said signals to the signal storage and display devices, thus allowing to reduce the rate of information to be transmitted and the consumption of the device.

Differential modulation techniques such as for example Continuously Variable Slope Delta-modulation (CVSD), widely used for voice transmission in Bluetooth devices, are used for compressing the digitized signal.

The measurement and arrangement of the mentioned nanotubes incorporated in the body of the sensor, projecting therefrom grouped like a brush, is such that they can penetrate the stratum corneum, the last layer of the skin, thus offering a better reception of the electrical signal

This new arrangement for the capturing means of the sensor allows:
1. Preventing the need to scrape the skin of the area chosen for capturing data, thus reducing the time used in the measurement and the discomfort caused to the patient.
2. Dispensing with an interface substance or gel, thus eliminating the noise introduced in the measurement caused by said interface substance or gel and also decreasing the time used in the measurement.

In other words, by using the electrophysiological sensor provided by the present invention, a preparation of the skin before or after transmitting electrical signals through the skin will not be required.

Said nanotubes can likewise be incorporated in an array placed or fixed in different supports such as a garment, a pillow or a mattress, being able to go unnoticed by the patient and not interfering in the measurements taken. Furthermore, the actual fabrics will also be able to have integrated communication infrastructures.

An electrophysiological sensor as described herein can be used in communication in the body area by using weak and therefore safe electrical signals. In other words, using the inside of the body as a conductor for transferring information for the so-called Body network area, including communications with implants.

According the present invention, the use of a technology of carbon nanotubes is highly interesting due to the fact that said nanotubes are extremely small, thus increasing health safety by decreasing the risk of infection. The nanotubes used are good conductors, inert and extremely hard and consistent.

According to an embodiment, a reasonable value for the resistance of the assembly of carbon nanotubes has to be less than 100kΩ; given that the impedance of a multiple wall carbon nanotube, which are those used herein, is 1000kΩ, it is sufficient for a few to penetrate the skin or establish a low impedance electrical contact by means of another mechanism (capacitance). From this data and carrying out the appropriate calculations the conclusion is reached that the sensor provided by the present invention will consists of at least 20 nanotubes considering that all of them have full contact with the skin.

### Brief Description of the Drawings

The previous and other advantages and features of the electrophysiological sensor of the invention will be more fully understood from the following detailed description of the attached drawings, in which:
Figure 1 shows an assembly of carbon nanotubes (CNT) according to the invention applied directly to the skin, it being observed that said nanostructure penetrates the stratum corneum, the last layer of the skin and also observing the cover of the sensor and the installation for transmitting the signal schematically;
Figure 2 shows a second embodiment similar to the first in which an amplifier installed inside the cover of the assembly has been added to the sensor; and
Figure 3 shows an electrical diagram or equivalent circuit representative of the operation of the sensor described by the present invention, the resistance of the sensor, the properties of the interface and other variables being indicated in this depiction;

### Detailed Description of Several Embodiments

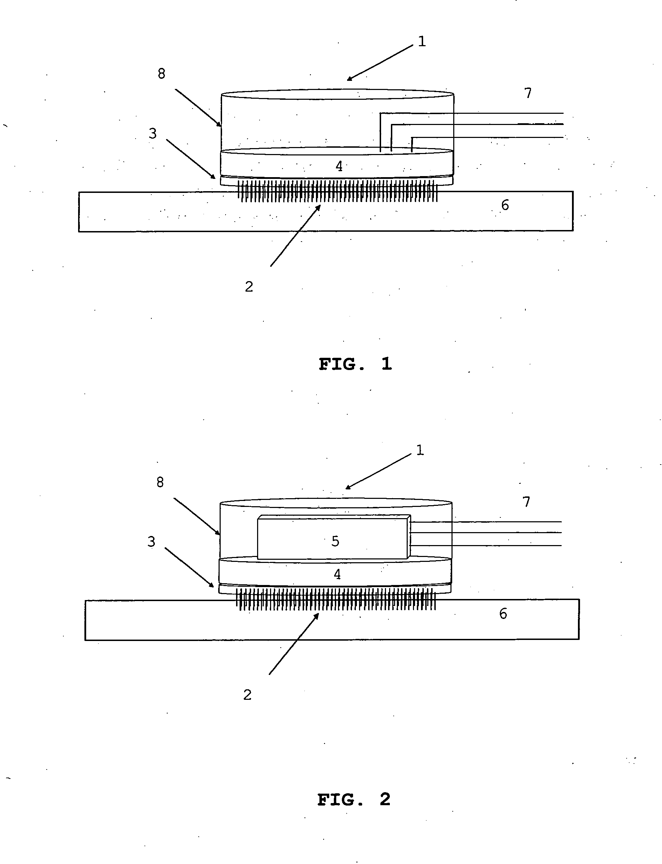

The electrophysiological sensor 1 according to the invention is integrated by a casing 8 housing an assembly of carbon nanotubes (CNT) 2 supported directly on a conducting substrate 3 from which they emerge like very fine rigid needles, suitable for being able to directly come into contact with the skin 6, without applying an electrolytic gel, and at least partially penetrate the stratum corneum, the outermost layer of the skin, as observed in Figure 1. Figure 1 has also detailed an electrical connector 4 which is used as a bridge between the conducting substrate 3 and an installation or at least one wiring 7 for transporting the signal to a remote point.

With the electrophysiological sensor 1 presented by the present invention provided with a structure of inert (i.e., non-polarizable) carbon nanotubes 2, in the form of rigid filiform elements grouped like a brush which can penetrate said outer layer of the skin, a transmission of electrical signals carrying information is achieved, from an organic tissue, with less noise with respect to the sensors of the state of the art and without needing an intermediate interface layer. Said structure of carbon nanotubes (CNT) 2 is further designed so that it penetrates very little, without coming into contact with the nerve cells, thus preventing any sensation of pain in the patient or possible transmission of an infection.

Advantageously the carbon nanotubes 2 used have multiple walls whereby their conductivity is increased and the capture and transmission of the biological potential signals is favored.

The mentioned multiple wall carbon nanotubes 2 have been obtained by growing directly in the substrate 4, which is made of highly doped silicon or titanium.

In another embodiment shown in Figure 2 the sensor 1 additionally includes a signal amplification system 5 integrated within the actual casing 8 in order to decrease the noise in the measurements. In this case the previously described arrangement of elements is reproduced again, there is an interface of CNT 2 in the form of bristles of a brush in contact with the outermost layer of the skin 6 (slightly penetrating therein) and there is also an installation or transport means 7, such as a wiring for transporting the electrical signal, which this time will be connected directly to the amplifier device 5.

The sensor of the invention can be formed such that it can be directly incorporated into different substrates, such as garments, pillows, mattresses or others. In a particular embodiment it has been provided that said conducting substrate is integrated in a material forming part of a surface of said garment or other article providing a substrate.

Taking into account that the electrophysiological sensor of the invention operates as a biological electrode such as a transducer converting an ionic current into an electrical current, it has been provided that in a possible embodiment the sensor of the invention also uses a suitable coating for facilitating the Red-Ox reaction in the interface area and for such purpose the mentioned nanotubes 2 are at least partially coated (in the region of their tips), with a suitable coating and particularly with an Ag-AgCl coating.

In another implementation of the invention it has been provided that the described electrophysiological sensor further houses an electronic circuit for processing and treating the signal, and a radio-frequency transmitter.

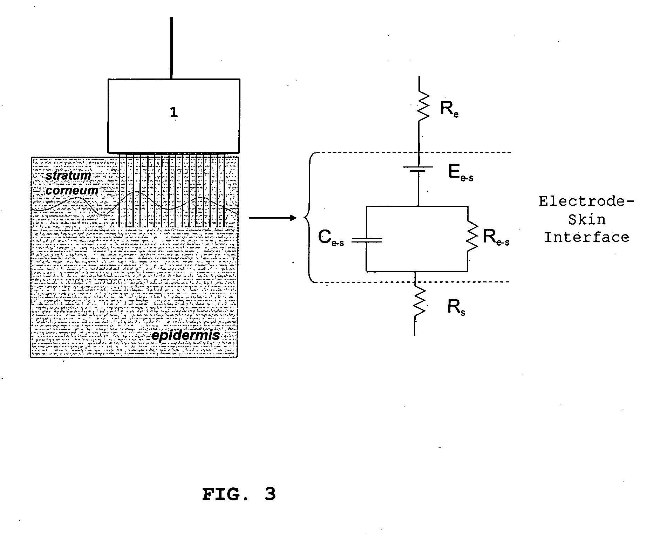

Figure 3 shows a simulation by means of an equivalent circuit of the performance of the electrophysiological sensor of the invention, in relation to an ionic signal captured by the sensor.

Unlike the performance of a similar sensor of the state of the art in which there will be at least two RC couplings, with their corresponding associated output and input resistances, corresponding respectively to a first gel-skin interface and to a second gel-electrode interface, in the proposal of the present invention said RC coupling is limited to the skin-sensor coupling, a considerable reduction in the noise introduced in the captured weak signal being derived therefrom due to the smaller charge accumulations in the interface layers, especially upon avoiding the gel-skin interface which is known to provide a component of instability most probably due to the diffusion of the gel in the stratum corneum which is a dynamic nonhomogeneous process.

Among the several applications for the electrophysiological sensor of the invention, the substitution of known electrophysiological electrodes for EEG, ECG, EMG, EOG, or for brain-machine interface applications, as well as the use of said sensor for communications between devices in the human body (external or implants) using the inside of the body as a conducting means, must be emphasized.

Other applications of the described electrophysiological sensor are in the area of research on sleep and particularly for monitoring the wakefulness or sleep state in an individual or for fatigue studies and in the area of biometry based on EEG and ECG.

A person skilled in the art could introduce changes and modifications in the embodiments described and introduce other elements in the sensor capturing chain, without departing from the scope of the invention as it is defmed in the attached claims.

## Claims

1. An electrophysiological sensor of the type which is based on conducting nanostructures which can transmit an electrical signal captured from the skin or from another part of an organic tissue to a transmitter means (4, 7) for transmitting said signal, **characterized in that** it comprises a plurality of said nanostructures which adopt a rigid filiform configuration and are chemically connected at one end to a conducting substrate (3) electrically coupled to said transmitter means, said nanostructures being operable to at least partially penetrate, by their free end, in said organic tissue or skin (6).

2. The electrophysiological sensor according to claim 1, **characterized in that** said plurality of nanostructures in the form of rigid filiform elements comprise conductive and inert carbon nanotubes (2) grouped like bristles of a brush.

3. The electrophysiological sensor according to claim 1, **characterized in that** said carbon nanotubes (2) have multiple walls.

4. The electrophysiological sensor according to any of the previous claims, **characterized in that** said conducting substrate (3) is made of highly doped silicon or titanium.

5. The electrophysiological sensor according to any of claims 1 to 4, **characterized in that** said nanotubes are at least partially coated with a coating facilitating a Red-Ox reaction.

6. The electrophysiological sensor according to any of claims 1 to 4, **characterized in that** said nanotubes are coated, in the region of their tips, which can come into contact with the skin, with a coating facilitating a Red-Ox reaction.

7. The electrophysiological sensor according to any of claims 1 to 4, **characterized in that** said nanotubes are at least partially coated with an Ag-AgCl coating.

8. The electrophysiological sensor according to any of claims 1 to 4, **characterized in that** it integrates an envelopment casing (8) housing said transmitter means for transmitting said signal which comprises a connector (4) which is continued in a conductor or wiring (7), the casing (8) having arranged in an outer wall said conducting substrate (3) supporting the nanostructures (2).

9. The electrophysiological sensor according to any of claims 1 to 4 or 8, **characterized in that** it integrates a local amplifier (5) in association with said transmitter means (4, 7).

10. The electrophysiological sensor according to any of claims 1 to 4 or 8, **characterized in that** it is incorporated in different supports, such as garments, pillows, mattresses or others.

11. The electrophysiological sensor according to any of claims 1 to 4 or 8, **characterized in that** it is incorporated in different supports, such as garments, pillows, mattresses or others, and **in that** said conducting substrate (3) is integrated in a material forming part of a surface of said garment or other article providing a substrate.

12. The electrophysiological sensor according to any of claims 1 to 4, **characterized in that** it further houses an electronic circuit for processing and treating the signal.

13. The electrophysiological sensor according to any of claims 1 to 4, **characterized in that** it further integrates a local amplifier (5) and a digital electronic circuit for controlling amplification.

14. The electrophysiological sensor according to any of claims 1 to 4, **characterized in that** it further integrates a digital electronic circuit adapted for compressing the digitized signals prior to transmitting the signal.

15. The electrophysiological sensor according to any of claims 1 to 4, **characterized in that** it further houses a wireless electronic circuit for transmitting data.

16. An use of an electrophysiological sensor according to any of claims 1 to 9 and 12 to 15, in EEG, ECG, EMG, EOG, or for brain-machine interface applications, biometric applications or systems for detecting fatigue and hypovigilance.

17. An use of an electrophysiological sensor according to any of claims 1 to 9 and 12 to 15, for monitoring the wakefulness or sleep state in an individual.
